# FASCICULE DE BREVET EUROPEEN

(11) **EP 1 467 724 B1**
(45) Date de publication et mention de la délivrance du brevet: **03.05.2006**
(21) Numéro de dépôt: 03731733.6
(22) Date de dépôt: 22.01.2003
(51) Int. Cl.: A61K 31/165, A61K 9/20

(54) **COMPOSITION PHARMACEUTIQUE ORODISPERSIBLE D'AGOMELATINE**
IN DER MUNDHÖHLE DISPERGIERBARE PHARMAZEUTISCHE ZUSAMMENSETZUNG MIT AGOMELATIN
ORODISPERSIBLE PHARMACEUTICAL COMPOSITION COMPRISING AGOMELATINE

(30) Priorité: 23.01.2002 FR 0200792
(43) Date de publication de la demande: 20.10.2004
(73) Titulaire: Les Laboratoires Servier, 92415 Courbevoie Cédex (FR)
(72) Inventeur: WUTHRICH, Patrick, F-45000 Orleans (FR); ROLLAND, Hervé, F-45160 Olivet (FR); JULIEN, Marc, F-45110 Sigloy (FR); THARRAULT, François, F-45000 Orléans (FR)
(86) Numéro de dépôt international: PCT/FR2003/000197
(87) Numéro de publication internationale: WO 2003/061644

(56) Documents cités:
- EP-A- 0 192 080
- EP-A- 0 447 285
- EP-A- 0 745 382
- EP-A- 1 175 899
- WO-A-97/42941

## Description

La présente invention a pour objet une forme pharmaceutique orodispersible solide pour l'administration par voie orale d'agomélatine.

L'agomélatine ou *N*-[2-(7-méthoxy-1-naphthyl)éthyl]acétamide est un agoniste sélectif des récepteurs mélatoninergiques.

L'agomélatine peut être administré par voie orale sous forme de comprimés à libération immédiate à avaler avec un demi-verre d'eau. Ces comprimés d'agomélatine sont utiles, notamment pour le traitement de la dépression, les troubles du sommeil et l'ensemble des pathologies liées à un dérèglement des rythmes circadiens.

Des études pharmacocinétiques chez l'homme ont montré que la biodisponibilité de l'agomélatine par voie orale est très faible par rapport à la voie parentérale et varie considérablement pour un même individu et d'un individu à l'autre.

Aussi, la biodisponibilité faible de l'agomélatine ainsi que les variations des concentrations inter et intra-individuelles ont conduit à rechercher une nouvelle formulation permettant de remédier à ces inconvénients.

Les compositions pharmaceutiques de la présente invention permettent non seulement de remédier aux inconvénients connus de la forme orale à libération immédiate mais également de proposer un service médical rendu supérieur permettant notamment l'amélioration de la qualité de vie des patients.

La composition pharmaceutique orodispersible d'agomélatine présente l'avantage d'une obtention rapide de taux plasmatiques élevés en principe actif en évitant la métabolisation importante du principe actif par effet de premier passage hépatique.

La composition pharmaceutique orodispersible selon l'invention présente la particularité de ne nécessiter ni eau ni mastication au cours de son administration. Elle se désagrège très rapidement dans la bouche, de préférence en moins de trois minutes et de manière encore plus préférentielle en moins d'une minute. Elle est administrée de façon préférentielle mais non exclusive sous la langue.

De nombreuses formes à dissolution rapide sont décrites dans l'art antérieur. De manière générale, les technologies décrites précédemment ont en commun l'utilisation d'un agent désintégrant comme le Kollidon® CL (polyvinylpyrrolidone réticulée), l'EXPLOTAB® (fécule carboxyméthylée), l'AC DISOL® (carboxyméthylcellulose sodique réticulée).

Cet agent de désintégration est indispensable dans la formulation des comprimés orodispersibles et doit être utilisé conjointement avec un excipient de compression directe. Les difficultés rencontrées pour la fabrication de tels comprimés résident dans le fait qu'il est très difficile d'obtenir des comprimés présentant des caractéristiques physiques constantes et reproductibles et compatibles avec les contraintes de manipulation classiques des comprimés.

En effet, les mélanges classiquement utilisés conduisent à des comprimés de dureté très élevée totalement inadaptée à une désagrégation rapide dans la cavité buccale.

D'autres formes orodispersibles sont réalisables par l'utilisation de la lyophilisation aboutissant à l'obtention de formes solides très poreuses dénommées "lyophilisat oral". Ces formes nécessitent l'utilisation d'un procédé industriel très spécifique, compliqué et long de mise en oeuvre, donnant une forme médicamenteuse à prix de revient élevé.

La présente invention permet de remédier à ces inconvénients. Elle concerne une forme solide orodispersible d'agomélatine contenant un excipient simple, d'origine naturelle permettant la désagrégation rapide, présentant une neutralité gustative et de texture agréable. Cet excipient joue le rôle à la fois de liant et de désintégrant. Il permet d'obtenir une formulation d'agomélatine simple, ayant une excellente aptitude à la compression directe conduisant à des comprimés de faible friabilité et de dureté compatible avec les techniques classiques de manipulation.

Plus particulièrement, l'invention concerne une composition pharmaceutique solide orodispersible d'agomélatine caractérisée en ce qu'elle contient :
- de l'agomélatine,
- et des granules consistant en lactose et amidon coséchés.

La composition selon l'invention peut également contenir, pour des raisons de fabrication, un ou plusieurs lubrifiants et un agent d'écoulement ainsi que des arômes, des colorants et des édulcorants, classiquement utilisés.

Pour des raisons d'amélioration de la tolérance locale de l'agomélatine (diminution de la sensation de picotements), l'agomélatine pourra éventuellement être associée à des excipients comme les cyclodextrines ou enrobée avec des excipients par l'utilisation de technologies connues de l'Homme de l'Art comme par exemple l'enrobage en lit d'air fluidisé, l'atomisation, la coacervation.

L'invention a également pour objet l'utilisation de granules consistant en lactose et amidon coséchés pour la préparation de compositions pharmaceutiques solides orodispersibles d'agomélatine.

On entend par le terme "orodispersible" des compositions pharmaceutiques solides qui se délitent dans la cavité buccale en moins de 3 minutes, et de préférence en moins d'une minute.

Lesdits granules compris dans les compositions pharmaceutiques solides selon l'invention correspondent aux compositions décrites dans la demande de brevet EP 00/402159.8. Ces granules sont caractérisés par une structure sphérique et une comprimabilité avantageuse et sont commercialisés sous l'appellation STARLAC®.

Les propriétés désintégrantes desdits granules sont connues pour des comprimés placés dans des volumes de liquides importants, sous agitation. Il est particulièrement surprenant que de tels granules employés pour la fabrication de formes orodispersibles puissent donner des résultats particulièrement satisfaisants en terme de désagrégation en bouche, et ce pour deux raisons.

La première est basée sur le constat que les excipients les moins solubles dans l'eau sont les plus appropriés à la formulation de comprimés orodispersibles (la solubilistion, entraînant une augmentation de viscosité de l'eau, est un frein à sa pénétration dans les comprimés). Or lesdits granules comprennent une fraction importante de lactose très soluble dans l'eau. De plus, l'amidon compris dans lesdits granules n'est pas un agent "super désintégrant" tel qu'utilisé et décrit dans les formes orodispersibles de l'art antérieur.

La deuxième est basée sur le constat que les propriétés de désintégration d'un excipient (utilisé dans un comprimé) évaluées dans l'eau par les méthodes conventionnelles ne sont pas extrapolables au comportement du même comprimé in vivo, dans la salive. En effet, les vitesses de désintégration dans l'eau sont mesurées (selon la Pharmacopée Européenne) dans une quantité d'eau suffisamment importante pour ne pas atteindre la saturation en terme de solubilisation, alors que in vivo, de par le faible volume de salive, les excipients sont à saturation. De plus, l'agitation à laquelle sont soumis les comprimés lors du test usuel ne reflète pas la désagrégation en bouche. La Demanderesse a ainsi constaté lors d'essais comparatifs que certains excipients connus comme bons désintégrants n'étaient pas adaptés à la préparation de formes orodispersibles. Inversement, certains excipients se désintégrant moyennement dans l'eau peuvent présenter des propriétés avantageuses in vivo.

La Demanderesse a alors trouvé que lesdits granules conféraient de façon surprenante aux comprimés de très bonnes aptitudes à se désagréger en bouche, et ce pour une large gamme de duretés de comprimés, tout en conservant une friabilité faible ce qui est particulièrement remarquable. En effet, la plupart des formes orodispersbiles de l'art antérieur qui se délitent rapidement dans la bouche sont très friables, ce qui se traduit par la nécessité d'utiliser un conditionnement spécifique et par des risque de désagrégation du comprimé dès qu'il est manipulé et ôté de son emballage.

Il est particulièrement remarquable que les critères d'orodispersibilité et de friabilité faible précités soient respectés pour une large gamme de dureté de comprimés, c'est-à-dire pour des comprimés présentant une dureté comprise entre 15 et 30 Newtons.

Les compositions pharmaceutiques selon l'invention sont préférentiellement caractérisées en ce qu'elles contiennent, par rapport au poids total du comprimé :
- de 0,2 % à 10 % en poids d'agomélatine.
- de 85 % à 98,5 % en poids de STARLAC®.

Elles contiendront éventuellement de 0,1 % à 3 % en poids d'agents lubrifiants comme le stéarate de magnésium ou le stéaryl-fumarate de sodium, préférentiellement de 0,5 % à 1,5 %, et de 0,1 % à 3 % en poids d'un agent d'écoulement comme la silice colloïdale, préférentiellement de 0,5 % à 1,5 %.

Les exemples suivants illustrent l'invention :
Comprimés orodispersibles d'agomélatine

### EXEMPLE 1 :

### Formulation : Comprimé terminé à 50 mg

| ***Constituants*** | ***Quantité (mg)*** |
|---|---|
| Agomélatine | 0,5 |
| Starlac® | 48,5 |
| Stéarate de magnésium | 0,5 |
| Silice colloïdale anhydre | 0,5 |

### EXEMPLE 2 :

### Formulation: Comprimé terminé à 100 mg

| ***Constituants*** | ***Quantité (mg)*** |
|---|---|
| Agomélatine | 5 |
| Starlac® | 93,5 |
| Stéarate de magnésium | 1 |
| Silice colloïdale anhydre | 0,5 |

Les comprimés sont préparés par mélange des constituants suivi d'une compression directe. La dureté des comprimés des exemples 1 et 2 est environ égale à 20 Newtons.

Afin d'évaluer le temps de désagrégation en bouche, les comprimés orodispersibles d'agomélatine décrits dans les exemples 1 et 2 ont été placés sous la langue pour favoriser le passage systémique par voie sublinguale de l'agomélatine et afin d'éviter au maximum l'effet de premier passage hépatique.

Lors de ces tests, il s'est avéré que pour chacune des formulations testées le temps de désagrégation dans la bouche était inférieur à 1 minute.

## Revendications

1. Composition pharmaceutique solide orodispersible d'agomélatine **caractérisée en ce qu'**elle comprend :
- de l'agomélatine,
- des granules consistant en lactose et amidon coséchés.

2. Composition pharmaceutique selon la revendication 1 **caractérisée en ce qu'**elle comprend, par rapport au poids total de la composition :
- de 0,2 % à 10 % en poids d'agomélatine.
- de 85 % à 98,5 % en poids de granules consistant en lactose et amidon coséchés.

3. Composition pharmaceutique selon la revendication 1 **caractérisée en ce qu'**elle comprend également un ou plusieurs lubrifiants, et un agent d'écoulement.

4. Composition pharmaceutique selon la revendication 1 **caractérisée en ce qu'**elle se présente sous forme de comprimé.

5. Comprimé selon la revendication 4 **caractérisé en ce qu'**il est obtenu par compression directe.

6. Comprimé selon la revendication 5 **caractérisé en ce que** sa dureté est comprise entre 15 et 50 Newtons.

7. Comprimé selon la revendication 6 **caractérisé en ce que** sa dureté est environ égale à 20 Newtons.

8. Utilisation de granules consistant en lactose et amidon coséchés dans la fabrication des compositions solides orodispersibles d'agomélatine se délitant en bouche en moins de trois minutes et de préférence en moins d'une minute.

9. Composition pharmaceutique solide orodispersible d'agomélatine, selon la revendication 1, utile pour le traitement de la dépression, des troubles du sommeil et de l'ensemble des pathologies liées à un dérèglement des rythmes circadiens.

## Patentansprüche

1. Feste, in der Mundhöhle dispergierbare pharmazeutische Zusammensetzung mit Agomelatin, **dadurch gekennzeichnet, daß** sie:
- Agomelatin und
- aus gemeinsam getrockneter Lactose und Stärke bestehende Körnchen umfaßt.

2. Pharmazeutische Zusammensetzung nach Anspruch 1, **dadurch gekennzeichnet, daß** sie, bezogen auf das Gesamtgewicht der Zusammensetzung:
- 0,2 % bis 10 Gew.-% Agomelatin und
- 85 % bis 98,5 Gew.-% der aus gemeinsam getrockneter Lactose und Stärke bestehenden Körnchen umfaßt.

3. Pharmazeutische Zusammensetzung nach Anspruch 1, **dadurch gekennzeichnet, daß** sie zusätzlich ein oder mehrere Gleitmittel und ein Fließverbesserungsmittel umfaßt.

4. Pharmazeutische Zusammensetzung nach Anspruch 1, **dadurch gekennzeichnet, daß** sie in Form einer Tablette vorliegt.

5. Tablette nach Anspruch 4, **dadurch gekennzeichnet, daß** sie durch direktes Verpressen erhalten worden ist.

6. Tablette nach Anspruch 5, **dadurch gekennzeichnet, daß** ihre Härte zwischen 15 und 50 Newton beträgt.

7. Tablette nach Anspruch 6, **dadurch gekennzeichnet, daß** ihre Härte etwa 20 Newton beträgt.

8. Verwendung von aus gemeinsam getrockneter Lactose und Stärke bestehenden Körnchen bei der Herstellung von festen, in der Mundhöhle dispergierbaren pharmazeutischen Zusammensetzungen mit Agomelatin, die sich in weniger als drei Minuten, vorzugsweise weniger als einer Minute, im Mund zersetzen.

9. Feste, in der Mundhöhle dispergierbare pharmazeutische Zusammensetzung mit Agomelatin nach Anspruch 1, für die Behandlung von Depressionen, Schlafstörungen, der Gesamtheit von pathologischen Zuständen, die mit einer Störung der 24-Stunden-Rhythmen verbunden sind.

## Claims

1. Solid orodispersible pharmaceutical composition of agomelatine, **characterised in that** it comprises:
- agomelatine,
- granules consisting of co-dried lactose and starch.

2. Pharmaceutical composition according to claim 1, **characterised in that** it comprises, in relation to the total weight of the composition:
- from 0.2 % to 10 % by weight of agomelatine,
- from 85 % to 98.5 % by weight of granules consisting of co-dried lactose and starch.

3. Pharmaceutical composition according to claim 1, **characterised in that** it also comprises one or more lubricants and a flow agent.

4. Pharmaceutical composition according to claim 1, **characterised in that** it is in the form of a tablet.

5. Tablet according to claim 4, **characterised in that** it is obtained by direct compression.

6. Tablet according to claim 5, **characterised in that** its hardness is from 15 to 50 Newtons.

7. Tablet according to claim 6, **characterised in that** its hardness is about 20 Newtons.

8. Use of granules consisting of co-dried lactose and starch in the manufacture of solid orodispersible compositions of agomelatine which disintegrate in the mouth in less than three minutes, preferably less than one minute.

9. Solid orodispersible pharmaceutical composition of agomelatine, according to claim 1, for use in the treatment of depression, sleep disorders and all pathologies associated with deregulation of circadian rhythms.
